(19) Europäisches Patentamt European Patent Office Office européen des brevets

(11) **EP 4 046 599 A1**

## (12) EUROPEAN PATENT APPLICATION
published in accordance with Art. 153(4) EPC

(43) Date of publication:
**24.08.2022 Bulletin 2022/34**

(21) Application number: **20877644.3**

(22) Date of filing: **19.08.2020**

(51) International Patent Classification (IPC):
**A61F 2/16** (2006.01)       **A61L 27/14** (2006.01)

(86) International application number:
**PCT/KR2020/011051**

(87) International publication number:
**WO 2021/075697 (22.04.2021 Gazette 2021/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.10.2019 KR 20190130027**

(71) Applicant: **Losec Co., Ltd.
Gyeonggi-do 16006 (KR)**

(72) Inventor: **PARK, Kyung Jin
Seoul 06232 (KR)**

(74) Representative: **Fabiano, Piero
Fabiano, Franke & MGT Sagl
Piazzetta San Carlo, 2
6900 Lugano (CH)**

(54) **INTRAOCULAR LENS**

(57)     Proposed is an intraocular lens capable of improving a change in refractive power of the lens by receiving transferred movement of the zonule of Zinn. The present invention is an intraocular lens inserted into the capsular sac, the intraocular lens consisting of an optic portion and a haptic portion, wherein the optic portion includes a front membrane, a rear membrane, and an internal space. The shape of the optic portion changes by the haptic portion receiving transferred external movement. The optic portion has additional regulatory power due to a curvature difference between the inner surfaces of the front membrane and the rear membrane, and thus provides a lens having a wide range of refractive power.

Fig. 5

Processed by Luminess, 75001 PARIS (FR)

EP 4 046 599 A1

**Description**

**Technical Field**

The present invention relates to an intraocular lens provided in a capsule sac.

**Background Art**

As a treatment method for ophthalmic diseases with abnormalities in the eye's lens, such as cataracts, an operation to remove the lens contents inside the capsular sac and insert an artificially manufactured intraocular lens into the space where the original lens sat is now commonly performed worldwide.

When the intraocular lens is inserted, the intraocular lens can replace the natural lens and provide a patient with unclouded vision. The problem with the conventional intraocular lens insertion procedure is that an anterior capsule and a posterior capsule of the capsule sac adhere with each other after surgery, and the original function of adjusting the thickness of the lens by transmitting relaxation and contraction of the zonule of Zinn is lost.

In other words, after surgery, the patient's eye does not secure a field of vision by performing an active three-dimensional movement according to an object to be seen, but a passive vision is secured according to the power determined by the intraocular lens.

As for the improved adjustable intraocular lens, even if the thickness of the lens can be adjusted, the degree of change is small so the effect of correcting vision is not great, and the range of accommodation is narrow since only twodimensional movement among the movements of the zonule of Zinn is reflected, and thus there is a limitation in that the improved adjustable intraocular lens may be applied only to patients over the age of 45 who have poor accommodative ability of the eye.

**Disclosure**

**Technical Problem**

Accordingly, the present invention is proposed to solve the above-described problems and an objective of the present invention is to provide an intraocular lens with improved accommodative power that can convert the three-dimensional complex movement received from a haptic portion into a change in refractive power of the lens.

According to an embodiment of the present invention, there is provided an intraocular lens of a new principle, in which a fluid in an internal space is not connected to the outside of an optic portion, that does not adjust the thickness of the optic portion by applying hydraulic pressure, and that does not have a separate adjusting means for optic portion thickness.

**Technical Solution**

According to an embodiment of the present disclosure for achieving the objective as described above, provided is an intraocular lens, including:

an optic portion configured to be inserted into an eyeball,
wherein the optic portion includes:
a front membrane facing outward direction of a gaze of the eyeball;
a rear membrane forming an internal space with the front membrane therebetween; and
a junction part where edges of the front membrane and the rear membrane are joined,
wherein the front membrane and the rear membrane are provided with a predetermined thickness and have inner and outer surfaces, respectively,
wherein the internal space is an enclosed space surrounded by the inner surface of the front membrane and the inner surface of the rear membrane and filled with a flowable material therein, and a shape of which is changed according to accommodation of the front membrane and the rear membrane.

The internal space may include: a central space formed in a center of the front membrane and the rear membrane, and convexly formed; and
a peripheral space formed at a periphery of the front membrane and the rear membrane, and in which a distance between the front membrane and the rear membrane is reduced to form the junction part.

Also, a first region of the front membrane and the rear membrane may be formed at a position corresponding to the central space,

wherein in the first region, it is preferable that a curvature of the inner surface of the front membrane is greater than the curvature of an outer surface of the front membrane, the curvature of the inner surface of the rear membrane is greater than the curvature of the outer surface of the rear membrane, and the outer surfaces of the front membrane and the rear membrane include spherical surfaces in a cross-sectional view of the intraocular lens cut vertically.

A second region of the front membrane and the rear membrane may be formed at a position corresponding to the peripheral space,

In the second region, it is preferable that the curvature of the inner surface of the front membrane is greater than or equal to the curvature of the outer surface of the front membrane, the curvature of the inner surface of the rear membrane is greater than or equal to the curvature of the outer surface of the rear membrane, and the inner surfaces of the front membrane and the rear membrane are aspherical surfaces in the cross-sectional view of the intraocular lens cut vertically.

It is preferable that the front membrane,

in the cross-sectional view of the intraocular lens cut vertically,

forms a curve with a convex surface, wherein the curve includes the spherical surface having a first radius of curvature R in the first region, and forms as the aspherical surface having a larger radius of curvature than the first radius of curvature R in the second region.

It is preferable that the aspherical surface having a radius of curvature greater than the first radius of curvature R has a radius of curvature that continuously increases toward the junction part.

Here, at a boundary between the junction part and the second region, the curvature of the inner surface of the front membrane may be 1 to 1.05 times the curvature of the outer surface of the front membrane, and the curvature of the inner surface of the rear membrane may be 1 to 1.05 times the curvature of the outer surface of the rear membrane.

The junction part may be integrally formed, and the junction part may be an extension of the front membrane and the rear membrane to be bonded.

In addition, the intraocular lens may further include at least one haptic portion provided on a circumferential surface of the optic portion, and the haptic portion may further include a connector provided and fixed in the eyeball or a connecting means for connecting to a support.

Also, the connecting means may be a female groove provided in the connector or the support and a male protrusion provided at an end of the haptic portion.

It is preferable that a volume of the internal space is 5 to 50 vol% of the optic portion.

It is preferable that the front membrane and the rear membrane are made of a material having a refractive index of 1.336 or more, preferably made of a material having a refractive index of 1.45 or more.

It is preferable that the flowable material has a refractive index of 1.336 or more, preferably 1.4 or more.

In addition, materials of the front membrane and the rear membrane may include any one or more of silicone, silicone elastomer, silicone polymer, polydimethyl siloxane, polyimide, polybutester, microplex PMMA, acrylic, flexible acrylic, hydrophobic acrylic, UV absorbing acrylate, methacrylate copolymer, butyl acrylate, polysiloxane elastomer, UV absorbing polysiloxane, collagen copolymer, cellulose acetate butylate (CAB), n-vinyl pyrrolidone (NVP), polyvinyl pyrrolidone (PVP), methacrylic acid (MA), glycerol methacrylate (GMA), dimethyl siloxane (DMS), polyethylene glycol methyl methacrylate (PEGMMA), and silicone hydrogels.

The flowable material may be made of a material containing silicone fluid, a substance having a silicone hardness of 10 or less, or methyl methacrylate (MMA).

Two or more haptic portions may be included so that the lens can be deformed in a single axial direction to correct astigmatism.

Another aspect of the present invention is a manufacturing method for an intraocular lens,

the method including: preparing an intermediate to prepare the intraocular lens intermediate including an optic portion and a haptic portion;

forming an internal space to form the internal space in a central part of the optic portion; and

injecting to inject a flowable material into the internal space.

The optic portion includes:

a front membrane facing outward direction of a gaze of an eyeball;

a rear membrane forming the internal space with the front membrane therebetween; and

a junction part where edges of the front membrane and the rear membrane are joined.

It is preferable that the front membrane and the rear membrane are provided with a predetermined thickness and have inner and outer surfaces, respectively,

the internal space is an enclosed space surrounded by the inner surface of the front membrane and the inner surface of the rear membrane and filled with the flowable material therein, and a shape of which is changed according to accommodation of the front membrane and the rear membrane, and
the forming an internal space is a step in which a laser is used for processing or forming the internal space.

Also, it is preferable that in the forming an internal space, the internal space forms: a central space formed in a center of the front membrane and the rear membrane and convexly formed toward an outside; and a peripheral space formed at a periphery of the front membrane and the rear membrane and in which a distance between the front membrane and the rear membrane is reduced.

The front membrane may include: a spherical surface having a first radius of curvature R in a first region corresponding to the central space; and an aspherical surface having a radius of curvature greater than the first radius of curvature R in a second region corresponding to the peripheral space.

In the forming an internal space, the internal space may be formed in a ratio of 5 to 50 vol% of the optic portion.

It is preferable that the method further further includes a step of finishing to remove an injection means used after the injecting, and to dispose of a track left by the injection means.

**Advantageous Effects**

As described above, the intraocular lens according to the embodiment of the present invention provides the effect of improving the ability to adjust the shape of the optic portion by the complex movement generated from the ciliary muscle and transmitted through the zonule of Zinn and capsular sac.

The inner surfaces of the front and rear membranes of the intraocular lens, according to the embodiment of the present invention, include a shape having a greater curvature than the outer surface at the center of the optic portion, and have better shape deformation ability than the case where the outer surfaces have a larger curvature than the inner surfaces at the center of the optic portion, so additional accommodative power can be obtained.

In addition, a separate connection part configuration may not be included since the fluid in the internal space does not require complicated movement, so the durability of the intraocular lens can be improved.

When using the intraocular lens of a preferred embodiment of the present invention as an intraocular lens assembly in combination with a support and a connecting means, the intraocular lens support transmits the complex motion of the zonule of Zinn on the outer circumferential surface, and the intraocular lens produces a change in refractive power from the complex motion through the connecting means.

The complex motions such as torsion, including motions in the X-axis, Y-axis, and Z-axis directions of the zonule of Zinn, are accurately transmitted to the intraocular lens through the haptic portion, creating a slight difference in refractive power, as a result, the function of the intraocular lens is improved, and the effect of correcting astigmatism can be obtained by adjusting the number and direction of the connecting means.

The intraocular lens according to an embodiment of the present invention provides large range of accommodation, so the intraocular lens can be used for vision correction for all age groups, not just limited to elderly patients with poor accommodative ability of the eye, thereby expanding the scope of application of the technology.

**Description of Drawings**

FIG. 1 is a cross-sectional view showing a human eyeball;
FIG. 2 is a cross-sectional view explaining the structure of a natural crystalline lens;
FIG. 3 is a cross-sectional view of an intraocular lens according to an embodiment of the present invention;
FIG. 4 is a cross-sectional view of the intraocular lens according to the embodiment of the present invention inserted into a capsular sac in combination with a support and a connecting means;
FIG. 5 is a cross-sectional view of the intraocular lens according to the embodiment of the present invention inserted into a capsular sac in combination with a support and a connecting means;
FIG. 6 is a cross-sectional view of an intraocular lens according to another embodiment of the present invention inserted into a capsular sac in combination with a support and a connecting means;
FIG. 7 is a state diagram showing the interaction and movement state of the zonule of Zinn and the lens in near viewing situation;
FIG. 8 is a state diagram showing the interaction and movement state of the zonule of Zinn and the lens in far viewing situation; and
FIG. 9 is a perspective view showing the connecting means of the intraocular lens.

**Best Mode**

Here 1) the shape, size, ratio, angle, and number, etc. shown in the accompanying drawings are schematic and may be slightly changed. 2) Since the drawing is shown from an observer's point of view, the direction or position for explaining the drawing may be variously changed according to the position of the observer. 3) Even if the reference numbers are different, the same reference numbers may be used for the same part.

4) When "include, have, consist of" is used, other parts may be added unless "only" is used. 5) If it is described in the singular, it can also be interpreted as the plural. 6) Even if the shape, size comparison, and positional relationship, etc. are not described as "approximately or substantially", it is interpreted to include the normal error range.

7) Even if terms such as "after, before, then, subsequently, and at this time" are used, they are not used in the meaning of limiting the temporal location. 8) Terms such as "first, second, and third" are simply used selectively, interchangeably, or repeatedly for the convenience of classification and are not to be interpreted in a limiting sense.

9) When the positional relationship of the two parts is described as "on, on the top, on the bottom, next to, on the side, and between", etc., one or more other parts may be placed between two parts unless "right" is used.

10) When parts are electrically connected with "~ or", it is interpreted to include not only the parts alone but also the combination, when electrically connected as "or, one of", it should be interpreted as only the parts alone.

11) Accommodation means different types of state in which adjustable intraocular lenses are to control refractive power. The state in which the refractive power is increased for near vision is called the compressed state or the accommodated state, and the state in which the refractive power is decreased for distant vision is called an uncompressed state or unaccommodated state. Before being inserted into the eye, the intraocular lens is in the uncompressed or unaccommodated state. In this specification, the contents representing or describing the shape of the intraocular lens are interpreted according to the situation based on the uncompressed state or unaccommodated state.

12) Diopter is a unit of measurement of the refractive power of a lens or a curved mirror, and it is equal to the reciprocal of the focal length measured in meters. Diopter is mainly used to indicate the the eye's power and power of lens. Generally, diopter value varies depending on the material of the lens and the relative refractive index, but in the air, it has a positive (+) value for a convex lens and a negative (-) value for a concave lens.

13) An optic portion is the part that performs optical functions in the intraocular lens, and it collects light entering the eye and forms an image on the retina like a lens.

14) A haptic portion is the part that performs the role of preventing movement and tilting by fixing the optic portion inside the capsular sac so that the optic portion can function stably in the intraocular lens. In the specification, when the intraocular lens is coupled with a connector or a support, it is interpreted to include a structure that further includes a connecting means coupled with a connector or a support.

15) When the term "aspherical" is used, it means a curved surface that is not spherical or flat on any surface of an object, and it refers to a form that includes a surface whose radius of curvature is not constant, which is used when calculating the degree of curvature of a curved surface. A shape in which the radius of curvature increases in an aspherical surface means a shape that is not a spherical surface, such as a parabola, a hyperbola, and a part of an ellipse, but increases as the radius of curvature corresponding to each section proceeds along the arc of the curve.

16) When the term 1st radius of curvature R is used, it does not mean a constant as a specific radius of curvature value, but is interpreted as a concept meaning an appropriate value of one of the radius of curvature of any curved surface.

17) The main meridian means the meridian in the direction that determines the axis of astigmatism, and the meridian refers to the poles on the extraocular surface connecting the anterior pole of the anterior center of the cornea and the posterior pole of the posterior center of the eyeball.

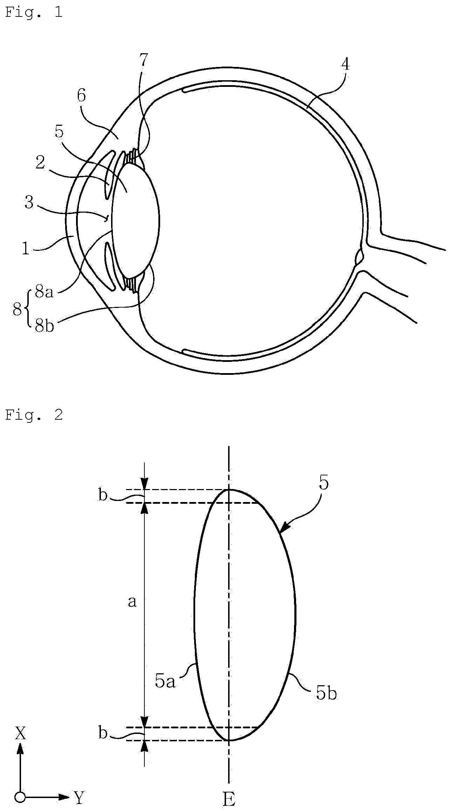

FIG. 1 is a cross-sectional view showing a human eyeball. The intraocular lens is an artificial crystalline lens that is inserted into the capsular sac 8, serves as the eye's natural crystalline lens 5 located behind the pupil, and has a mainly convex lens shape.

FIG. 2 is a cross-sectional view showing a human eye lens. The natural lens has a shape in which the front 5a has a smaller curvature than the rear 5b in the central part.

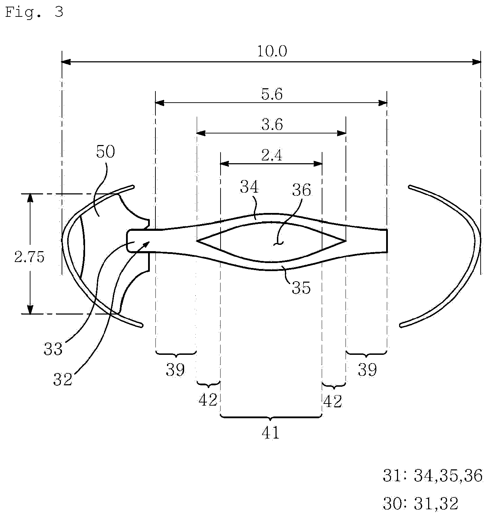

FIG. 3 is a cross-sectional view of an intraocular lens according to an embodiment of the present invention. The intraocular lens 30 is composed of an optic portion 31 positioned at the rear of the pupil, and a haptic portion 32 radially protruding from the optic portion 31 and fixed to a connecting means, etc. The optic portion 31 includes a front membrane 34, a rear membrane 35, and an internal space 36. The front membrane 34 and the rear membrane 35 are divided based on the equator or equatorial plane of the optic portion. The front membrane 34 and the rear membrane 35 may meet at a peripheral surface or a peripheral part of the optic portion 31, and may be directly connected or connected with another member or adhesive material. It is preferable that the front membrane 34 and the rear membrane 35 are made of the same material and are integrally formed.

The optic portion 31 is a part that performs a function similar to the actual eye lens by collecting incoming light to form an image. The haptic portion 32 is located on the periphery of the optic portion and is a means for supporting and fixing the optic portion 31, preferably providing a portion coupled to the connecting means, and may perform the function of transmitting the movement of the capsular sac to the optic portion 31.

The internal space 36 is an enclosed space located between the front membrane 34 and the rear membrane 35. The movement of the material present in the internal space 36 is limited as the internal space exists within a predetermined radius of the optic portion.

The front membrane 34 is positioned in the visual axis direction (Y direction) of the optic portion when the intraocular lens is inserted, and is inserted into the eyeball to face outward direction of a gaze of the eyeball. The front membrane 34 may be made of a material having a refractive index of 1.336 or more, preferably 1.40 or more, and more preferably 1.45 or more.

The front membrane 34 may have a shape that is thin at the center and becomes thicker or maintained toward the equator. The front membrane 34 may have a negative diopter value in some regions including the center. Also, inner surface of the front membrane includes a region having a greater curvature than the outer surface of the front membrane.

The rear membrane 35 is positioned in the opposite direction to the visual axis direction (Y direction) of the optic portion when the intraocular lens is inserted. The rear membrane 35 may be made of a material having a refractive index of 1.336 or more, preferably 1.40 or more, and more preferably 1.45 or more. The rear membrane 35 may be manufactured integrally by selecting the same material as the front membrane 34. Materials of the front membrane and the rear membrane may include any one or more of silicone, silicone elastomer, silicone polymer, polydimethyl siloxane, polyimide, polybutester, microplex PMMA, acrylic, flexible acrylic, hydrophobic acrylic, UV absorbing acrylate, methacrylate copolymer, butyl acrylate, polysiloxane elastomer, UV absorbing polysiloxane, collagen copolymer, cellulose acetate butylate (CAB), n-vinyl pyrrolidone (NVP), polyvinyl pyrrolidone (PVP), methacrylic acid (MA), glycerol methacrylate (GMA), dimethyl siloxane (DMS), polyethylene glycol methyl methacrylate (PEGMMA), and silicone hydrogels.

The rear membrane 35 may have a negative diopter value in some regions including the center. Also, the inner surface of the the rear membrane includes a region having a greater curvature than the outer surface of the the rear membrane. Due to a difference in curvature between the inner surface and the outer surface, the rear membrane 35 may have a shape that is thin at the center and becomes thicker or maintained toward the equator.

The internal space 36 is an enclosed space surrounded by the inner surfaces of the front membrane 34 and the rear membrane 35. In an embodiment of the present invention, the shape of the internal space coincides with the shape formed by the inner surfaces of the front membrane 34 and the rear membrane 35, and even if the shape changes, the volume can be maintained without accompanying fluid flow with the outside. The internal space 36 is filled with a flowable material. The flowable material may have a refractive index less than or equal to that of the material of the front membrane 34 or the rear membrane 35, and preferably a material having the same refractive index as the material of the front membrane and the rear membrane.

When the refractive index of the material constituting the front membrane 34 is A, the refractive index of the material constituting the rear membrane 35 is B, and the refractive index of the flowable material filling the internal space 36 is C, C is less than or equal to the lesser greater of A and B. Preferably, A and B have the same value, and C may be less than or equal to this value.

The internal space 36 includes a shape that is thick at the center and becomes thinner or maintained toward the equator, and it includes a shape, even if this shape is different from the shape just mentioned, which allows the entire optic portion 31 to have the same effect as the effect of the present invention. When the refractive indices of the materials of the front membrane 34, the rear membrane 35 and the internal space 36 are all the same, the refractive index in the unaccommodated state may not be limited by the shape of the internal space. The internal space 36 has a positive (+) diopter value at the center of the intraocular lens 30.

The internal space 36 may be made of a material having a refractive index of 1.336 or more, preferably 1.4 or more. The internal space 36 may be made of a material including silicone fluid, a material having a silicone hardness of 10 or less, or methyl methacrylate (MMA). Sodium hyaluronate, chondroitin sulfate, hydroxypropyl methylcellulose, polyacrylamide, etc. may be additionally included.

The outer surface of the front membrane is the outside of the front membrane 34 in the viewing direction or the visual axis direction. The outer surface of the front membrane is symmetrical to the central axis, includes a curved shape convex in the viewing direction, and has a shape including a spherical surface. The outer surface of the front membrane may include a spherical surface similar to the curved surface 5a corresponding to the shape of the actual eye lens shown in FIG. 2 or an aspherical surface having several curvatures in the unaccommodated state.

The inner surface of the front membrane is a surface close to the rear membrane 35 and symmetrical to the central axis, has a curved shape convex in the viewing direction, and includes a spherical surface in the unaccommodated state. The inner surface curvature of the front membrane 34 may decrease or be maintained from the central part of the front membrane toward the equatorial part of the intraocular lens.

The outer surface of the rear membrane 35 includes a curved shape convex in a direction opposite to the viewing direction. The outer surface of the rear membrane 35 is symmetrical to the central axis and includes spherical and aspherical surfaces in the unaccommodated state. The outer surface of the rear membrane 35 may be symmetrical to the outer surface of the front membrane 34, and may include a spherical surface having a greater curvature than the outer surface of the front membrane 34. The outer surface of the rear membrane 35 may be a curved surface asymmetrical to the outer surface of the front membrane 34 or a curved surface similar to the curved surface 5b corresponding to the shape of the actual eye lens.

The inner surface of the rear membrane 35 is a surface close to the front membrane 34 and symmetrical to the central axis, has a curved shape convex in a direction opposite to the viewing direction (Y direction), and includes a spherical surface in the unaccommodated state. The inner surface curvature of the rear membrane 35 may decrease or be maintained from the central part of the rear membrane toward the equator of the intraocular lens, and may be symmetrical with the inner surface of the front membrane 34.

The internal space 36 preferably occupies 5 to 50 vol% of the volume of the optic portion. When the volume of the internal space 36 is less than 5 vol%, the thickness of the internal space 36 becomes too small for easy shape change, and thus it is difficult to obtain additional refractive power. When the volume of the internal space 36 is greater than 50 vol%, the thickness of the front membrane 34 and the rear membrane 35 may become too small, and the refractive index of the entire optical portion may be lowered.

The volume ratio occupied by the internal space 36 may be determined differently depending on the material and refractive index of the front membrane 34 and the rear membrane 35. The volume ratio occupied by the internal space 36 may be determined differently depending on the refractive index of the flowable material. The volume ratio occupied by the internal space 36 may also be determined differently depending on the various conditions including a patient's accommodation or desired corrected vision. In addition, the curvature of each inner and outer surface is adjusted to obtain a constant diopter value throughout the optic portion without changing the direction.

Hereinafter, the optic portion 31 will be divided into a plurality of regions.

The first region 41 is a region including the central part of the optic portion, and is a region in which the inner surfaces of the front and rear membranes 34 and 35 and the outer surfaces of the front and rear membranes form a spherical surface or an aspherical surface close to the spherical surface. The eccentricity of the curved surfaces of the front membrane 34 and the rear membrane 35 of the first region 41 may be 0.1 or less, preferably 0.05 or less. The first region 41 may include a spherical surface having a first radius of curvature R.

The second region 42 is a region existing around the central part of the optic portion, and includes a front membrane 34 and the rear membrane 35 in an area including the maximum diameter region of the internal space 36. The curved surfaces of the inner surfaces of the front and rear membranes and the outer surfaces of the front and rear membranes of the second region 42 include aspherical surfaces whose curvature radius increases toward the junction part 39. The curved surfaces of the inner surfaces of the front and rear membranes and the outer surfaces of the front and rear membranes of the second region 42 may include aspherical surfaces whose curvature decrease rate increases toward the junction part 39. The curved surfaces of the inner surfaces of the front and rear membranes and the outer surfaces of the front and rear membranes of the second region 42 form continuous curved surfaces with the spherical or aspherical surfaces of the adjacent first region 41 or the junction part 39.

The curvature of the inner surface of the front membrane at the boundary between the junction part and the second region is 1 to 1.05 times the curvature of the outer surface of the front membrane at the boundary between the junction part and the second region, and the curvature of the inner surface of the rear membrane at the boundary between the junction part and the second region is 1 to 1.05 times the curvature of the outer surface of the rear membrane at the boundary between the junction part and the second region. It is preferable that the curvatures of the corresponding inner and the outer surfaces are the same.

An embodiment of the present invention is an intraocular lens in which the front and rear membranes are symmetrically configured. The inner surface of the front membrane has the largest curvature at the center and the smallest curvature at the boundary between the second region and the junction part. The curvature of the inner surface of the front membrane increases continuously from the center toward the junction part, though the rate of change of the curvature is not constant. That is, the closer to the center, the smaller the rate of change, and the closer to the junction part, the greater the rate of change. For this reason, the amount of change in the curvature of the inner surface of the front membrane in the first region is smaller than the amount of change in the curvature of the inner surface of the front membrane in the second region. Preferably, the amount of change in curvature in the first region may be within 0.3 to 0.5 times the amount of change in curvature in the second region. The curvature of the outer surface of the front membrane increases continuously from the center toward the junction part. The curvature at the boundary between the second region and the junction part may be 1 to 0.95 times the curvature of the inner surface of the front membrane at the corresponding position, or preferably the same. It is preferable that the curvature at the boundary between the second region and the junction part is maintained while the outer surface of the front membrane extends to the junction part.

The eccentricity of the curved surfaces of the inner surfaces of the front and rear membranes and the outer surfaces of the front and rear membranes of the second region 42 may be 0.1 or more, and may be 0.05 or more. The second region 42 includes an aspherical surface having a larger radius of curvature than the first radius of curvature R. Also, the second region 42 may not exist depending on the shape of the internal space 36.

The first region 41 and the second region 42 include front and rear surfaces corresponding to the region where the internal space 36 of the optic portion exists. In the first region 41 and the second region 42, the outer surfaces of the front membrane 34 and the rear membrane 35 include a curvature smaller than that of the inner surface to have a negative diopter value.

This difference in curvature between the inner surface and the outer surface causes the front membrane 34 and the rear membrane 35 to have a negative diopter in the region including the center, and to promote a shape change in the accommodated state to obtain additional accommodative power. The direction of the curvature does not change on the inner surfaces of the front membrane 34 and the rear membrane 35 and only the magnitude of the curvature changes to have a surface that curves outward. The additional accommodative power changes according to the difference in curvature of the outer and inner surfaces, and by adjusting this, an intraocular lens having the desired lens power and accommodative power may be obtained. When the direction of curvature on the inner surfaces of the front membrane 34 and the rear membrane 35 is changed, due to the high refractive index of the material constituting the front and rear membranes, the diopter value, which is the power of the lens, may not naturally follow in the section where the direction of curvature changes.

The junction part 39 is a peripheral region located outside the second region 42, is a region in which the front membrane 34 and the rear membrane 35 are extended to form an integral or joined part, and is a region in which the internal space 36 does not exist. The outer surface of the junction part 39 includes a spherical surface. At the end of the junction part 39, an aspherical surface may be included or the direction of curvature of the curved surface may be changed.

The optic portion 31 may be implemented to have a constant diopter value as a whole in the first region 41, the second region 42, and the junction part 39, or to have a constant diopter value in the first region 41 and the second region 42 and a low diopter value in the junction part 39.

The internal space 36 is an enclosed space formed by the inner surfaces of the front membrane 34 and the rear membrane 35, and has a central space 37 including the central part and including convex surfaces in the anterior and posterior directions and a peripheral space 38 located around the central part and in which the distance between the front membrane 34 and the rear membrane 35 is reduced.

The central space 37 is a region corresponding to the first region 41 of the front and rear membranes 34 and 35, has a surface that curves outward in the anterior and posterior directions, and has an additional accommodative power due to a shape that becomes thicker toward the peripheral space 38. The central space 37 has a curved shape including a spherical surface in some regions.

The peripheral space 38 is a region corresponding to the second region 42 of the front and rear membranes 34 and 35, and includes convex surfaces in the anterior and posterior directions. The peripheral space 38 is formed in the peripheral part of the front membrane 34 and the rear membrane 35, and in which the space between the front membrane 34 and the rear membrane 35 is reduced to form the junction part 39 where the front membrane and the rear membrane meet. The peripheral space 38 may not exist depending on the shape of the internal space 36.

The junction part 39 refers to a part where the front membrane 34 and the rear membrane 35 are joined or integrally formed, and is a region in which the internal space 36 and the inner surface do not exist. The front membrane 34 and the rear membrane 35 corresponding to the junction part 39 have only an outer surface. The junction part 39 may be formed by an adhesive suitable for the material of the front and rear membranes.

As shown in FIG. 3, an embodiment of the present invention has an internal space 36 having a maximum diameter of 3.6 mm, a section having a diameter of 2.4 mm or less is a first region 41, and has a second region 42 having a diameter of 2.4 mm to 3.6 mm. The outer and inner surfaces of the front membrane 34 form a spherical surface in a section with a diameter of 2.4 mm at the center, and an aspherical surface with an increasing degree of aspheric surface in a section with a diameter of 2.4 mm to 3.6 mm, and the curvature decreases toward the outside. The outer surface of the front membrane 34 includes a spherical surface in a section with a diameter of 3.6 mm or more. The second region 42 connects the spherical region of the first region 41 and the spherical region of the junction part 39, and the curvature of each region continuously changes.

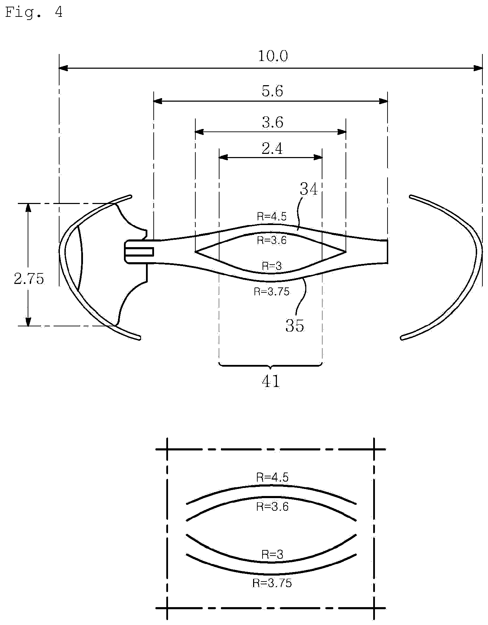

FIG. 4 is a cross-sectional view of an intraocular lens according to another embodiment of the present invention. The radius of curvature of each surface of the front membrane 34 and the rear membrane 35 of the intraocular lens is indicated in the figure in mm. In the center of the first region 41, the radius of curvature of the outer surface of the front membrane is 4.5 mm, and the radius of curvature of the inner surface of the front membrane is 3.6 mm. In the center of the first region 41, the radius of curvature of the outer surface of the rear membrane is 3.75 mm, and the radius of curvature of the inner surface of the rear membrane is 3.0 mm.

The radius of curvature of the inner surface is smaller than that of the outer surface in both the front membrane 34 and the rear membrane 35, so that the curvature of the curved surface is greater on the inner surface. In the relationship between the front membrane 34 and the rear membrane 35, the radius of curvature at the rear membrane is smaller and the curvature is greater.

Additionally, a part of the spherical curved surface according to the numerical value of the radius of curvature is shown in the lower section of the cross-sectional view of FIG. 4.

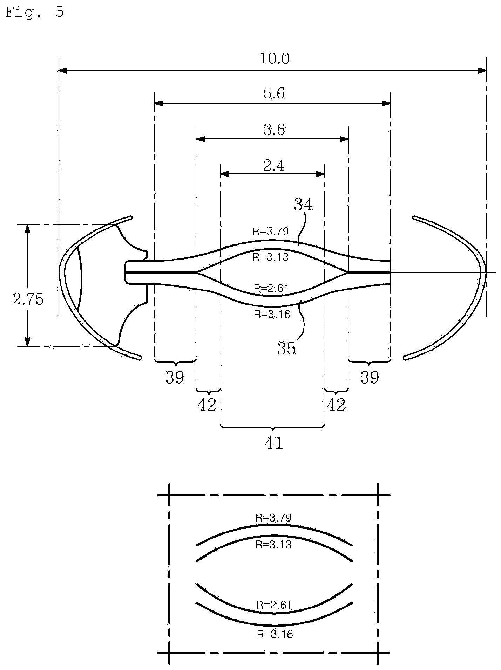

FIG. 5 is a cross-sectional view of an intraocular lens according to yet another embodiment of the present invention. In the center of the first region 41, the radius of curvature of the outer surface of the front membrane is 3.79 mm, and the radius of curvature of the inner surface of the front membrane is 3.13 mm. In the center of the first region 41, the radius of curvature of the outer surface of the rear membrane is 3.16 mm, and the radius of curvature of the inner surface of the rear membrane is 2.61 mm.

Additionally, a part of the spherical curved surface according to the numerical value of the radius of curvature of the embodiment is shown in the lower section of the cross-sectional view of FIG. 5.

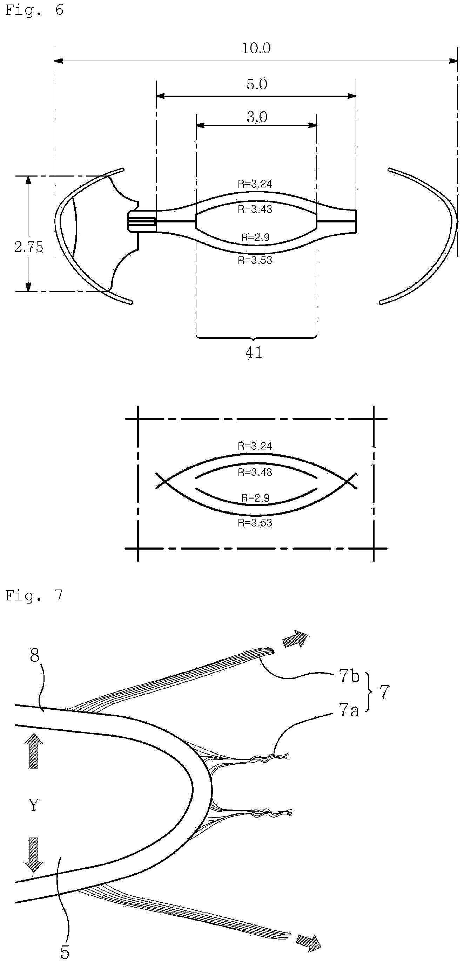

FIG. 6 is yet another embodiment of the present invention. The first region 41 is a region having a diameter of 3.0 mm or less, and the junction part is a region having a diameter of 3.0 mm to 5.0 mm or less. In the center of the first region 41, the radius of curvature of the outer surface of the front membrane is 3.24 mm, and the radius of curvature of the inner surface of the front membrane is 3.43 mm. In the center of the first region 41, the radius of curvature of the outer surface of the rear membrane is 3.53, and the radius of curvature of the inner surface of the rear membrane is 2.9 mm.

The intraocular lens has an internal space 36 having a maximum diameter of 3.0 mm and a section having a diameter of 3.0 mm or less is a first region 41, and does not include a second region. The outer and inner surfaces of the front membrane 34 form a spherical surface or an aspherical surface close to a spherical surface in a section with a diameter of 3.0 mm from the center, and the outer surface of the front membrane 34 includes a spherical or aspherical surface in a diameter of 3.0 mm to 5.0 mm.

In the second region 42, it is preferable that the decrease in curvature of the inner surface of the front membrane or the inner surface of the rear membrane increases as the distance from the center increases. For example, the amount of change in the curvature from the outer end of the second region 42 to the middle of the second region 42 may be more than three times the amount of change in curvature from the central end of the second region 42 to the middle of the second region 42.

An embodiment of the present invention determines the relationship between the width of the central space 37, the peripheral space 38, and the junction part 39 in the intraocular lens.

When the radius of the central space 37 is x (mm) and the width of the peripheral space 38 and the junction part 39 in a section cut in plane including the central axis is y and z (mm), respectively, it is preferable that x, y, z satisfy the following expression.

$$y * z \leq 1.6 + |x - 1.5| - x \; (However, when\; A \geq 0, |A|\; is\; A, when\; A < 0, |A|\; is\; -A)$$

In an embodiment, x=1.2, y=0.6, z=1.0, so when substituting into the above formula, the value of the left side is 0.6*1.0, which is 0.6, and the value of the right side is 1.6+0.3-1.2, so it becomes 0.7, and the inequality is established. In the case of an embodiment in which there is no surrounding space, since x=1.5, y=0, z=1.0, the value of the left side is 0 and the value of the right side is 0.1, so the inequality is established.

The haptic portion 32 includes a structure protruding outward from the circumferential surface of the optic portion 31. The shape of the haptic portion is not limited. The haptic portion may be radially directed outward from the circumferential surface of the optic portion, may include one or two or more protruding structures, and may be in the form of a disk, ring, tube, or torus. Preferably, three structures may be formed at intervals of 120 degrees.

When there are three or less haptic portions, it is easy to couple to the connecting means when inserting the intraocular lens. Even when there are four or more haptic portions, insertion may be facilitated when the intervals between the haptic portions are not uniformly arranged.

When there are two haptic portions, or even three or more are arranged to transmit a force to the optic portion along one axis passing through the center of the optic portion, by the received force, the optic portion is deformed into an oval or rugby ball shape with one axis as a minor axis to form a major meridian with maximum refractive power, thereby obtaining a correction effect for astigmatism.

When the two haptic portions are inserted into the eyeball, they may be arranged in the horizontal direction, so that the accommodative power of the capsular sac is transmitted to the intraocular lens in one axial direction to cause asymmetrical shape change. Even if there are four or more haptic portions, the distance formed by each haptic portion may not be uniformly arranged, but may be arranged to be deflected in the horizontal direction. In this case, similar to the case where there are two haptic portions, an asymmetrical shape change may be induced, so that an astigmatism correction effect can be obtained.

In addition, the end of the haptic portion 32 is coupled to an intraocular lens support or connector provided in the eyeball to fix and support the intraocular lens and receive movement of the capsular sac. At this time, the end of the haptic portion 32 may be connected by a connecting means, and the method and form of the connecting means are not limited.

In an embodiment of the present invention, the end or part of the haptic portion 32 has a male protrusion, and a connector and a support having a female groove coupled to the male protrusion are coupled.

**Mode for Invention**

In another embodiment of the present invention, the end of the haptic portion 32 has one or more grooves, and the connector or the support has a connecting means having a shape engageable with the grooves of the haptic portion.

When the connecting means is formed, the force for transmitting the movement of the zonule of Zinn may be more effectively transmitted to the intraocular lens 30 through the support and the haptic portion 32.

It is preferable that the haptic portion 32 has a protruding width greater than a protruding length from the peripheral part of the optic portion 31. When the protruding width is smaller than the protruding length, the force such as torsion acting in the three-axis direction may not be transmitted properly, so forming the protruding width greater than the protruding length is an important factor in improving the movement transmission performance. Also, it is preferable that the width of the haptic portion 32 increases as it protrudes.

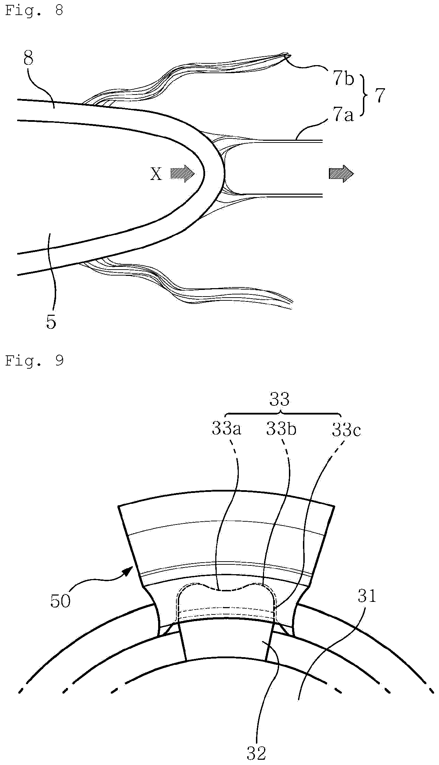

FIG. 9 is a perspective view showing a connection means according to an embodiment of the present invention. A fixing portion 33 is provided at the end of the haptic portion 32 as a connection means. In the embodiment, the fixing portion 33 is provided in a form in which a plurality of convex parts and a concave part are sequentially provided at the protruding end. The convex part 33b is provided on each side of the concave part 33a in the center of the fixing portion. A side part 33c in contact with the convex part 33b on both sides thereof has a curved shape. The fixing portion 33 is fixed in engagement with the shape of the groove of the connector 50 to support the optic portion 31 and the haptic portion 32.

Although not shown in FIG. 9, the concave part and the convex part may also be formed on the side part 33c. As the fixing portion 33 having the concave part and the convex part is provided at the end of the haptic portion 32 and coupled with the connecting means of the connector 50, movement transmission ability to transmit the force acting not only on the X and Y axes but also on the Z axis is improved.

That is, when the concave part 33a and the convex part 33b are not provided, there may be a loss of force due to slippage or the like. On the contrary, when the concave part 33a and the convex part 33b are provided, slipping may be prevented and the movement transmission ability may be improved.

The process in which the intraocular lens, which is a preferred embodiment of the present invention configured as described above, is used is as follows.

When the intraocular lens according to the embodiment of the present invention is coupled by an intraocular support and a connecting means to be used as an assembly, the state of each configuration is as follows.

As shown in FIG. 7, in far viewing situation, the first zonule 7a of the capsular sac 8 becomes taut and the second zonule 7b of the capsular sac 8 becomes loose. As a result, the equatorial part of the capsular sac 8 receives a force extending in the X direction, and the elastic intraocular lens 30 located inside the capsular sac 8 also stretches in the same direction so that its thickness is reduced or the position is changed.

At this time, the force applied by the zonule of Zinn 7 does not act only in one direction of the X axis, but actually causes a complex motion in which vectors in the three directions of X, Y, and Z are combined.

As shown in FIG. 8, in near viewing situation, the first zonule 7a of the capsular sac 8 becomes loose and the second zonule 7b of the capsular sac 8 becomes taut. As a result, the equatorial part of the capsular sac 8 receives a force extending in the Y direction, and the elastic intraocular lens 30 located inside the capsular sac 8 also stretches in the same direction so that its thickness is increased or the position is changed.

At this time, the force applied by the zonule of Zinn 7 does not act only in one direction of the Y axis, but actually causes a complex motion in which vectors in the three directions of X, Y, and Z are combined. Also, this compound motion (including torsion) is transmitted to the optic portion 31 of the intraocular lens 30 via the haptic portion 32.

The equatorial part of the optic portion 31 is compressed inward, and the shapes of the front membrane 34 and the rear membrane 35 made of a flexible material are deformed. The front membrane 34 has a structure in which the center is thinner than the periphery, so that it is displaced forward and has a greater curvature than before. The rear membrane 35 has a structure in which the center is thinner than the periphery, so that it is displaced backward and has a greater curvature than before.

The front membrane 34 and the rear membrane 35 are deformed to move away from each other, so that the thickness increases at the center of the internal space. The optic portion 31 is deformed to have a greater positive (+) diopter value than before the force is applied.

The intraocular lens according to the embodiment of the present invention is different from the existing hydraulically controlled intraocular lens because the fluid is not connected to the outside of the optic portion or actively moves to control the thickness of the optic portion by applying hydraulic pressure, and does not require a means for changing the thickness or distance of the front and rear membranes as an essential component.

The front membrane 34 and the rear membrane 35 of the intraocular lens according to another embodiment of the present invention have a structure in which the center is thinner than the periphery, which is made due to the difference in curvature between the inner surface and the outer surface at the center, and this structure makes it possible to easily change the shape of the front membrane 34 and the rear membrane 35 to provide additional accommodative power.

In an embodiment in which the curvature of the inner surface is greater than the curvature of the outer surface in the central part of the front membrane 34 and the rear membrane 35, the curvature deformation of the optic portion may occur more easily. Since the thickness change of the lens occurs greatly at the center of the optic portion 31, due to the difference in curvature between the inner and outer surfaces of the central part, a larger change in curvature can be expected in a structure in which the thickness of the central part is smaller than that of the periphery, resulting in additional accommodative power.

An embodiment of the present invention is an intraocular lens in which the front and rear membranes include a shape symmetrical to the equatorial part, and another embodiment is an intraocular lens in which the front and rear membranes include an asymmetrical shape similar to that of a natural lens.

Hereinafter, a method for manufacturing an intraocular lens which is another aspect of the present invention will be described. The manufacturing method according to an embodiment of this aspect includes: preparing an intermediate to prepare the intraocular lens intermediate including an optic portion and a haptic portion; forming an internal space to form the internal space in a central part of the optic portion; and injecting to inject a flowable material into the internal space.

The optic portion includes: a front membrane facing outward direction of a gaze of an eyeball; a rear membrane forming the internal space with the front membrane therebetween; and a junction part 39 where edges of the front membrane and the rear membrane are joined, wherein the front membrane and the rear membrane have inner and outer surfaces, respectively, wherein the internal space is surrounded by the inner surface of the front membrane and the inner surface of the rear membrane and a shape of which is changed according to accommodation of the front membrane and the rear membrane.

Preparing an intermediate is a step to prepare the intraocular lens intermediate including an optic portion and a haptic portion. In the preparing an intermediate, processing is made with a pre-selected material, and the front and rear membranes divided based on the equatorial plane of the optic portion are included. The same material may be selected for the front and rear membranes to be integrally formed, or different materials may be selected and manufactured and then joined.

In the preparing an intermediate, it is possible to manufacture an intraocular lens intermediate including an aspherical section in which the outer surface of the front membrane of the optic portion decreases in curvature toward the peripheral part, and including a spherical section continuously connected to the aspherical section.

In the preparing an intermediate, it is possible to manufacture the intraocular lens intermediate in which the outer surface of the front membrane forms a convex curve in the cross-sectional view of the intraocular lens cut vertically. Here, the convex curve includes a spherical surface in which the outer surface of the front membrane has a first radius of curvature R, and includes an aspherical surface having a greater radius of curvature than the first radius of curvature R in the periphery of the region formed in the central part.

The intraocular lens intermediate is made of a material having a refractive index of 1.336 or more, and is preferably made of a material having a refractive index of 1.45 or more.

The intraocular lens intermediate may be prepared including any one or more of silicone, silicone elastomer, silicone polymer, polydimethyl siloxane, polyimide, polybutester, microplex PMMA, acrylic, flexible acrylic, hydrophobic acrylic, UV absorbing acrylate, methacrylate copolymer, butyl acrylate, polysiloxane elastomer, UV absorbing polysiloxane, collagen copolymer, cellulose acetate butylate (CAB), n-vinyl pyrrolidone (NVP), polyvinyl pyrrolidone (PVP), methacrylic acid (MA), glycerol methacrylate (GMA), dimethyl siloxane (DMS), polyethylene glycol methyl methacrylate (PEGMMA), and silicone hydrogels.

Forming an internal space is a step to form an enclosed space formed by the inner surfaces of the front and rear membranes therein. When the front and rear membranes are made of the same material, the entire optic portion is integrated, so the inside may be directly processed without destroying the outside of the optic portion using a laser.

The internal space is a space formed in the center of the front and rear membranes, and includes a central space convexly formed and a peripheral space formed at a periphery of the front and rear membranes and in which a distance between the front membrane and the rear membrane is reduced to form the junction part.

In addition, the front membrane may be manufactured to include a spherical surface in the first region corresponding to the central space, and to include an aspherical surface with a radius of curvature greater than the radius of curvature of the spherical surface in the second region corresponding to the peripheral space.

When different materials are selected for the front membrane and the rear membrane, the front and rear membranes processed in the preparing an intermediate may be bonded to form the internal space. Alternatively, the front and rear membranes may be combined with the inside unprocessed, and then the internal space is formed using a laser.

In the forming an internal space, a step of introducing a removal means capable of discharging smoke and byproducts generated inside the optic portion to the outside of the optic portion may be additionally included.

In an embodiment of the present invention, same material is selected for the front and rear membranes, the internal space of the predetermined thickness and curvature is processed by a femtosecond laser in the forming an internal space, and a needle is used as a removal means to discharge smoke.

In the forming an internal space, it is preferable that the volume of the internal space is 5 to 50 vol% of the optic portion.

In the forming an internal space, the inner surfaces of the front membrane and the rear membrane may be processed to have a greater curvature than the corresponding outer surfaces in the first region, and to form a curved surface including a spherical surface, respectively. The inner surfaces of the front membrane and the rear membrane may be processed to form, in the second region 42, a curved surface including an aspherical surface having a curvature greater than or equal to that of the corresponding outer surfaces, respectively.

Injecting is a step to fill the internal space with a flowable material to have refractive power.

It is preferable that the flowable material has a refractive index that is less than or equal to the refractive index of the material constituting the intraocular lens intermediate, and is a material including silicon fluid, a substance with a silicone hardness of 10 or less, or methyl methacrylate (MMA). The flowable material may further include sodium hyaluronate, chondroitin sulfate, hydroxypropyl methyl cellulose, polyacrylamide, etc. The flowable material has a refractive index of 1.336 or more, preferably a material of 1.4 or more may be used.

The Intraocular lens may be manufactured, in which, in the cross-sectional view of the intraocular lens cut vertically, x, y, and z satisfy the following formula when the central space, the peripheral space, and the width of the junction part 39 are x, y, and z (mm), respectively.

$$y * z \leq 1.6 + |x - 1.5| - x \, (However, when \, A \geq 0, |A| \, is \, A, when \, A < 0, |A| \, is \, -A)$$

In an embodiment of the present invention, the injecting may involve manufacturing using an injection means, and the removal means of the forming an internal space step may be used as the injection means.

In another embodiment of the present invention, separating which is a step to remove the used injection means after the injecting step is included. Also, a step of finishing in which a track left by the injection means is naturally blocked of removed after the separating step may be included.

The finishing step may include removing the track by injecting an adhesive member into the track after the injection means is moved to a position between the inner and outer surfaces of the front or rear membranes. Here, the adhesive member is selected according to the material of the front and rear membranes, and when selecting an acrylic material, it is preferable to use an acrylic bond.

In yet another embodiment of the present invention, after the removal means is separated, the injecting step is performed using a new injection means, and after injecting step, the separating step to remove the injection means was performed. The track was fine so the finishing step was carried out without the use of an adhesive.

The present invention is not limited to the specific embodiments described above, various modifications can be made by anyone with ordinary skill in the art to which the present invention pertains without departing from the gist of the present invention as claimed in the claims, and such changes are within the scope of the claims.

[Description of Numerals]

5: lens 7: zonule of Zinn
8: capsular sac 30: intraocular lens
31: optic portion 32: haptic portion
33: fixing portion 34: front membrane
35: rear membrane 36: internal space
50: connector

**Claims**

1. An intraocular lens, comprising:

an optic portion configured to be inserted into an eyeball,
wherein the optic portion includes:

a front membrane facing outward direction of a gaze of the eyeball;
a rear membrane forming an internal space with the front membrane therebetween; and
a junction part where edges of the front membrane and the rear membrane are joined,
wherein the front membrane and the rear membrane are provided with a predetermined thickness and have inner and outer surfaces, respectively,
wherein the internal space is an enclosed space surrounded by the inner surface of the front membrane and the inner surface of the rear membrane and filled with a flowable material therein, and a shape of which is changed according to accommodation of the front membrane and the rear membrane.

2.  The intraocular lens of claim 1, wherein the internal space includes:

a central space formed in a center of the front membrane and the rear membrane, and convexly formed; and
a peripheral space formed at a periphery of the front membrane and the rear membrane, and in which a distance between the front membrane and the rear membrane is reduced to form the junction part.

3.  The intraocular lens of claim 2, wherein a first region of the front membrane and the rear membrane is formed at a position corresponding to the central space,
wherein in the first region, a curvature of the inner surface of the front membrane is greater than the curvature of an outer surface of the front membrane, the curvature of the inner surface of the rear membrane is greater than the curvature of the outer surface of the rear membrane, and the outer surfaces of the front membrane and the rear membrane include spherical surfaces in a cross-sectional view of the intraocular lens cut vertically.

4.  The intraocular lens of claim 3, wherein a second region of the front membrane and the rear membrane is formed at a position corresponding to the peripheral space,
wherein in the second region, the curvature of the inner surface of the front membrane is greater than or equal to the curvature of the outer surface of the front membrane, the curvature of the inner surface of the rear membrane is greater than or equal to the curvature of the outer surface of the rear membrane, and the inner surfaces of the front membrane and the rear membrane are aspherical surfaces in the cross-sectional view of the intraocular lens cut vertically.

5.  The intraocular lens of claim 4, wherein in the cross-sectional view of the intraocular lens cut vertically, the front membrane forms a curve with a convex surface,
wherein the curve includes the spherical surface having a first radius of curvature R in the first region, and forms as the aspherical surface having a larger radius of curvature than the first radius of curvature R in the second region.

6.  The intraocular lens of claim 5, wherein the aspherical surface having a radius of curvature greater than the first radius of curvature R has a radius of curvature that continuously increases toward the junction part.

7.  The intraocular lens of claim 6, wherein at a boundary between the junction part and the second region, the curvature of the inner surface of the front membrane is 1 to 1.05 times the curvature of the outer surface of the front membrane, and the curvature of the inner surface of the rear membrane is 1 to 1.05 times the curvature of the outer surface of the rear membrane.

8.  The intraocular lens of claim 7, wherein the junction part is integrally formed.

9.  The intraocular lens of claim 7, wherein the junction part is an extension of the front membrane and the rear membrane to be bonded.

10.  The intraocular lens of claim 8 or 9, further comprising:
at least one haptic portion provided on a circumferential surface of the optic portion.

11.  The intraocular lens of claim 10, wherein the haptic portion further includes a connector provided and fixed in the eyeball or a connecting means for connecting to a support.

12.  The intraocular lens of claim 11, wherein the connecting means is a female groove provided in the connector or the support and a male protrusion provided at an end of the haptic portion.

13. The intraocular lens of claim 12, wherein a volume of the internal space is 5 to 50 vol% of the optic portion.

14. The intraocular lens of claim 13, wherein the front membrane and the rear membrane are made of a material having a refractive index of 1.336 or more, preferably made of a material having a refractive index of 1.45 or more.

15. The intraocular lens of claim 14, wherein the flowable material has a refractive index of 1.336 or more, preferably 1.4 or more.

16. The intraocular lens of claim 15, wherein materials of the front membrane and the rear membrane include any one or more of silicone, silicone elastomer, silicone polymer, polydimethyl siloxane, polyimide, polybutester, microplex PMMA, acrylic, flexible acrylic, hydrophobic acrylic, UV absorbing acrylate, methacrylate copolymer, butyl acrylate, polysiloxane elastomer, UV absorbing polysiloxane, collagen copolymer, cellulose acetate butylate (CAB), n-vinyl pyrrolidone (NVP), polyvinyl pyrrolidone (PVP), methacrylic acid (MA), glycerol methacrylate (GMA), dimethyl siloxane (DMS), polyethylene glycol methyl methacrylate (PEGMMA), and silicone hydrogels.

17. The intraocular lens of claim 16, wherein the flowable material is made of a material containing silicone fluid, a substance having a silicone hardness of 10 or less, or methyl methacrylate (MMA).

18. The intraocular lens of claim 17, wherein two or more haptic portions are included so that the lens can be deformed in a single axial direction to correct astigmatism.

19. A manufacturing method for an intraocular lens, the method comprising:

preparing an intermediate to prepare the intraocular lens intermediate including an optic portion and a haptic portion;
forming an internal space to form the internal space in a central part of the optic portion; and
injecting to inject a flowable material into the internal space.

20. The manufacturing method for an intraocular lens of claim 19, wherein the optic portion includes:

a front membrane facing outward direction of a gaze of an eyeball;
a rear membrane forming the internal space with the front membrane therebetween; and
a junction part where edges of the front membrane and the rear membrane are joined,
wherein the front membrane and the rear membrane are provided with a predetermined thickness and have inner and outer surfaces, respectively,
wherein the internal space is an enclosed space surrounded by the inner surface of the front membrane and the inner surface of the rear membrane and filled with the flowable material therein, and a shape of which is changed according to accommodation of the front membrane and the rear membrane.

21. The manufacturing method for an intraocular lens of claim 20, wherein the forming an internal space is a step in which a laser is used for processing or forming the internal space.

22. The manufacturing method for an intraocular lens of claim 21, wherein in the forming an internal space, the internal space forms:

a central space formed in a center of the front membrane and the rear membrane and convexly formed toward an outside; and
a peripheral space formed at a periphery of the front membrane and the rear membrane and in which a distance between the front membrane and the rear membrane is reduced,
wherein the front membrane includes:

a spherical surface having a first radius of curvature R in a first region corresponding to the central space; and
an aspherical surface having a radius of curvature greater than the first radius of curvature R in a second region corresponding to the peripheral space.

23. The manufacturing method for an intraocular lens of claim 22, wherein in the forming an internal space, the internal space is formed in a ratio of 5 to 50 vol% of the optic portion.

24. The manufacturing method for an intraocular lens of claim 23, the method further comprising:
finishing to remove an injection means used after the injecting, and to dispose of a track left by the injection means.

Fig. 1

Fig. 2

Fig. 3

31: 34,35,36
30: 31,32

Fig. 4

10.0

5.6

3.6

2.4

34

R=4.5

R=3.6

R=3

R=3.75

35

2.75

41

R=4.5

R=3.6

R=3

R=3.75

Fig. 5

Fig. 6

Fig. 7

Fig. 8

Fig. 9

## INTERNATIONAL SEARCH REPORT

| International application No. |
| --- |
| **PCT/KR2020/011051** |

**A. CLASSIFICATION OF SUBJECT MATTER**

*A61F 2/16*(2006.01)i; *A61L 27/14*(2006.01)i

According to International Patent Classification (IPC) or to both national classification and IPC

**B. FIELDS SEARCHED**

Minimum documentation searched (classification system followed by classification symbols)

A61F 2/16; A61F 2/14; A61F 9/008; A61L 27/14

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 안구내렌즈(intraocular lens), 옵틱부(optic part), 햅틱부(haptic part), 전막(front plane), 후막(back plane), 곡률(curvature), 비구면(aspheric plane), 유동성소재(fluid), 실리콘(silicon), 메틸 메타크릴레이트 (methyl methacrylate)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2014-036770 A (HOYA CORP.) 27 February 2014. See paragraphs [0014]-[0128]; and figures 1-2. | 1 |
| Y | | 2-24 |
| Y | KR 10-0807939 B1 (PARK, Kyung Jin) 28 February 2008. See paragraphs [0050]-[0176]; and figures 3-4. | 2-24 |
| Y | KR 10-2016-0140279 A (LOSEC CO., LTD.) 07 December 2016. See paragraphs [0065]-[0069]; and figures 5-9. | 11-18 |
| X | US 2018-0177639 A1 (LENSGEN, INC.) 28 June 2018. See paragraphs [0002]-[0097]; claims 1-20; and figures 1A-16. | 1-2,19-21 |
| Y | | 3-18,22-24 |

☑ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **30 November 2020** | **30 November 2020** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2020/011051**

**C.    DOCUMENTS CONSIDERED TO BE RELEVANT**

| Category* | Citation of document, with indication, where appropriate, of the relevant passages | Relevant to claim No. |
|---|---|---|
| | US 2016-0128826 A1 (LENSGEN, INC.) 12 May 2016. See paragraphs [0007]-[0075]; and figures 1-6. | |
| X | | 1-2,19-21 |
| Y | | 3-18,22-24 |
| | US 2007-0021831 A1 (CLARKE, G. P.) 25 January 2007. See paragraphs [0011]-[0091]; and figures 1-8. | |
| X | | 1-2,19-21 |
| Y | | 3-18,22-24 |
| | US 2009-0319040 A1 (KHOURY, E.) 24 December 2009. See paragraphs [0032]-[0089]; and figures 1-12. | |
| X | | 1-2,19-21 |
| Y | | 3-18,22-24 |

Form PCT/ISA/210 (second sheet) (July 2019)

**INTERNATIONAL SEARCH REPORT**
Information on patent family members

International application No.

**PCT/KR2020/011051**

| Patent document cited in search report | | | Publication date (day/month/year) | Patent family member(s) | | | Publication date (day/month/year) |
|---|---|---|---|---|---|---|---|
| JP | 2014-036770 | A | 27 February 2014 | JP | 2014-036769 | A | 27 February 2014 |
| | | | | WO | 2014-027689 | A1 | 20 February 2014 |
| KR | 10-0807939 | B1 | 28 February 2008 | CN | 101636127 | A | 27 January 2010 |
| | | | | EA | 016270 | B1 | 30 March 2012 |
| | | | | EA | 200901215 | A1 | 30 April 2010 |
| | | | | EP | 2131785 | A1 | 16 December 2009 |
| | | | | EP | 2131785 | A4 | 02 November 2011 |
| | | | | JP | 05091259 | B2 | 05 December 2012 |
| | | | | JP | 2010-520011 | A | 10 June 2010 |
| | | | | US | 08603166 | B2 | 10 December 2013 |
| | | | | US | 2010-0016963 | A1 | 21 January 2010 |
| | | | | WO | 2008-108525 | A1 | 12 September 2008 |
| KR | 10-2016-0140279 | A | 07 December 2016 | EP | 3305250 | A1 | 11 April 2018 |
| | | | | EP | 3305250 | A4 | 13 March 2019 |
| | | | | JP | 06595102 | B2 | 23 October 2019 |
| | | | | JP | 2018-519981 | A | 26 July 2018 |
| | | | | JP | 2019-130376 | A | 08 August 2019 |
| | | | | KR | 10-1718075 | B1 | 04 April 2017 |
| | | | | US | 10758339 | B2 | 01 September 2020 |
| | | | | US | 2018-0147049 | A1 | 31 May 2018 |
| | | | | WO | 2016-195143 | A1 | 08 December 2016 |
| US | 2018-0177639 | A1 | 28 June 2018 | WO | 2013-166068 | A1 | 07 November 2013 |
| US | 2016-0128826 | A1 | 12 May 2016 | CN | 105073066 | A | 18 November 2015 |
| | | | | EP | 2934383 | A1 | 28 October 2015 |
| | | | | EP | 2934383 | A4 | 27 July 2016 |
| | | | | US | 09186244 | B2 | 17 November 2015 |
| | | | | US | 10111745 | B2 | 30 October 2018 |
| | | | | US | 2014-0180403 | A1 | 26 June 2014 |
| | | | | WO | 2014-099630 | A1 | 26 June 2014 |
| US | 2007-0021831 | A1 | 25 January 2007 | AU | 2006-270040 | A1 | 25 January 2007 |
| | | | | AU | 2006-270040 | B2 | 29 November 2012 |
| | | | | CA | 2615825 | A1 | 25 January 2007 |
| | | | | CA | 2615825 | C | 17 November 2015 |
| | | | | CA | 2902676 | A1 | 25 January 2007 |
| | | | | CA | 2902676 | C | 30 August 2016 |
| | | | | EP | 1909699 | A2 | 16 April 2008 |
| | | | | US | 08038711 | B2 | 18 October 2011 |
| | | | | US | 08475529 | B2 | 02 July 2013 |
| | | | | US | 2012-0035724 | A1 | 09 February 2012 |
| | | | | US | 2013-0231741 | A1 | 05 September 2013 |
| | | | | WO | 2007-011879 | A2 | 25 January 2007 |
| | | | | WO | 2007-011879 | A3 | 07 June 2007 |
| US | 2009-0319040 | A1 | 24 December 2009 | EP | 2056749 | A1 | 13 May 2009 |
| | | | | US | 08070806 | B2 | 06 December 2011 |
| | | | | WO | 2008-031231 | A1 | 20 March 2008 |

Form PCT/ISA/210 (patent family annex) (July 2019)